(19) Europäisches Patentamt
European Patent Office
Office européen des brevets

(11) **EP 3 718 508 B1**

(12) **EUROPEAN PATENT SPECIFICATION**

(45) Date of publication and mention of the grant of the patent:
**26.07.2023 Bulletin 2023/30**

(21) Application number: **19168659.1**

(22) Date of filing: **11.04.2019**

(51) International Patent Classification (IPC):
**B29C 45/43** (2006.01)   **A61F 2/12** (2006.01)
**B29L 31/00** (2006.01)

(52) Cooperative Patent Classification (CPC):
**A61F 2/12; B29C 45/435;** A61F 2240/001;
A61F 2240/004; B29L 2031/7532

(54) **MOULD AND MOULDING METHOD FOR BODY IMPLANT, IN PARTICULAR FOR BREAST IMPLANTS**

FORM UND FORMVERFAHREN FÜR KÖRPERIMPLANTATE, INSBESONDERE FÜR BRUSTIMPLANTATE

MOULE ET PROCÉDÉ DE MOULAGE POUR IMPLANT CORPOREL, EN PARTICULIER POUR DES IMPLANTS MAMMAIRES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.04.2019 PT 2019115423**

(43) Date of publication of application:
**07.10.2020 Bulletin 2020/41**

(73) Proprietor: **Celoplas - Plásticos Para A Industria S.A.**
**4775-127 Grimancelos, Barcelos (PT)**

(72) Inventors:
• **de Oliveira Cortez, João**
**4760-221 Vila Nova de Famalicão (PT)**
• **Rebelo Cortez, Ana Alexandra**
**4760-169 Vila Nova de Famalicão (PT)**

(74) Representative: **Patentree**
**Edificio Net**
**Rua de Salazares, 842**
**4149-002 Porto (PT)**

(56) References cited:
WO-A1-01/10525          WO-A1-97/18076
FR-A1- 2 734 754        US-A- 5 141 581
US-A1- 2003 134 067     US-A1- 2013 171 288

## Description

## TECHNICAL FIELD

The present disclosure relates to the moulding of flexible implant shells suitable for holding a filling material for flexible body implants, in particular flexible breast implants.

## BACKGROUND

It is known that due to a disease, accident or because aesthetic purposes, a medical device acting like a foreign body can be implanted in the human or animal body to modify the appearance of or replace a part of a body, by means of plastic surgery.

According the part of the body to be replaced or reconstructed, these foreign bodies can be implants or prosthesis.

In particular, flexible implants can be used to replace or reconstruct soft tissues and be totally placed inside the body requiring specific biocompatible materials, shapes, surface characteristics and mechanical performance.

Regarding breast implants, they can be inserted in the body by several techniques from under the pectoral muscle to subcutaneously, always looking for the less invasive technique.

Typically these implants are made of a biocompatible flexible shell, containing different quantities of a filling material that confers the desired volume and consistency to the implant.

The major state of the art for the production of these implants is by dipping, which consists on the immersion of a mould in a liquefied Polydimethylsiloxane batch. This method is typically done manually, which does not guarantee the necessary reproducibility of the method, as well as a precise control of the shell thickness.

A problem present in the state of the art is related to the biodurability of breast implant shells, which can be influenced by many factors, such as, material ageing, damage of the implant during surgical procedure, trauma injuries, open and closed capsulotomy, manufacturing defects and others.

The most frequent manufacturing defects, for example shell rupture, are mostly due to low reproducibility of the actual manufacturing methods, where high variations on mechanical behaviours can be found over different implant brands, but also over different batches of the same implant brand.

An existing solution to this problem, besides the aforementioned dipping technique, is to use cast moulding techniques, like dipping, or vacuum moulding process.

The document FR2734754, to which the preamble of claims 1 and 13 relates, discloses a process for the fabrication of an implantable surgical prosthesis having a hollow flexible tubular body from a polymerisable biocompatible material such as a silicone, comprising moulding the body in the form of a single flexible membrane which may exhibit variations in wall thickness, by vacuum casting a biocompatible material into a mould having an internal cavity which corresponds in form to the external shape of the body and which has a micro-textured surface, and which contains a core which defines the internal shape of the body, positioned in the mould by a key to define a casting cavity, whereby the cross section of the key at its point of entry into the cavity defines a demoulding aperture for removing the body from the core.

The document WO 97/18076 discloses a method for stripping a resilient bellows part from an injection mould comprising a step of injection moulding a resilient bellows part around a colinear core pin and a stripper rod, surrounded by a split cavity. The bellows part has two open ends, one of which is substantially rigid and is closed by the stripper rod passing through it. The second open end is resilient. Another step comprises opening the split cavity to permit the bellows part to be stripped from the core pin. Yet another step comprises expanding the bellows part radially outward from the core pin. Simultaneously with expanding the bellows part, a step of pushing the substantially rigid open end of the bellows part by the stripper rod causes the resilient portion and resilient open end to be stripped off the core pin. Finally, the substantially rigid open end of the bellows part is ejected from the stripper rod.

Document US2013171288 describes a system for moulding elastomeric breast implant shells. In the process of demoulding to cause disengagement between the moulded silicone elastomeric coating and the mandrel, a fluid flow of low pressure air is provided through a fluid port exposed to an inside surface of the moulded silicone shell.

However, the state of the art solutions are not able to compete with injection moulding on productivity, product cost, method reproducibility, design flexibility and dimensional tolerances.

Document US2003134067 describes a method and a mould for injection moulding of flexible polymeric envelopes. In the process of demoulding the moulded product is ejected without splitting or tearing using gas injection to expand the moulded product.

These facts are disclosed in order to illustrate the technical problem addressed by the present disclosure.

**GENERAL DESCRIPTION**

The invention is defined by a mould and a moulding method according to claims 1 and 13, respectively. A mould according to the present invention is characterised by the features recited in the characterising portion of claim 1. A moulding method for moulding according to the present invention is characterised by the features recited in the characterising portion of claim 13. The present disclosure includes a method of moulding flexible implant shells for medical purposes, which comprises the use of an injection mould and a fully automatic process that, after the entrance of an elastomeric material in the mould and its curing, allows the ejection of the final moulded shell automatically in particular by the radial inflation caused by a gas. The advantages thereof include ejection without causing any defects like rupture, outstanding controlled thickness distribution across the flexible implant shell and high production rates, considering that this mould design allows one or more mould cavities.

Thus, the present disclosure includes a method of moulding flexible implant shells for medical purposes which comprises an injection mould and a fully automated process that, after the entrance of an elastomeric material in the mould and its curing, allows the ejection of the final moulded shell automatically, in particular by the radial inflation caused by a gas.

The disclosed method allows the fabrication of flexible shells for medical purposes with better mechanical performance and lower risk of rupture due to a highly controlled thickness distribution, which can comply with tighter thickness tolerances allowing less probability of shell rupture.

The mould design can include more than one single cavity, leading to the possibility to produce more than one flexible shell in a single step, allowing a higher productivity and lower product cost.

The incorporation of a vacuum system on the mould allows to remove air and gases released by the chemical reaction of curing from the cavity, or cavities, improving the quality of flexible shells and improving thickness control, without any microvoids.

Embodiments of the disclosure allows ideal temperature distribution across the mould segments, comprising correct local thermal isolation to assure the correct temperature performance and monitoring, allowing the correct, quick cure of the mouldable material inside the mould and higher thermal stability.

The mechanical control of the ejection system comprising a radial entrance of air on the cavity allows the inflation of the moulded flexible shell by a gas, which is supported by a small mechanical displacement of an internal dynamic stem, which allows the automatic ejection from the core, without creating localized weak regions on the shell because of elongation that can lead to future rupture.

The presence of a shaft sealant between the static and the dynamic stem, allows the linear displacement of the dynamic stem inside the static stem, ensuring no ejection gas is lost.

It is disclosed a mould for injection moulding a flexible implant shell for use in body implants, comprising:

a mould core having a stalk and a convex bulbous element; and
two mould halves, wherein each mould half is arranged for mating with the other mould half at respective confronting surfaces, each surface comprising a concavity forming, in the mould halves, a mould cavity for mating with the bulbous element;
wherein said mould core comprises:

a first stem forming a first portion of the stalk and a proximal portion of the convex bulbous element;
a second stem forming a second portion of the stalk and a distal portion of the convex bulbous element;

arranged such that one of the stems is displaceable relative to the other stem for opening a gap at the bulbous element, between the first stem and the second stem, for injecting an ejection gas for ejecting the moulded shell.

It should be understood that the feature that one of the stems is displaceable relative to the other stem comprises the situations where a first stem is moveable, i.e. dynamic, and the other stem is fixed, i.e. static, where a first stem is fixed, i.e. static, and the other stem is moveable, i.e. dynamic, or where both stems are moveable, i.e. dynamic; such that a relative displacement between the stems is able to open a gap between the stems, at the bulbous element, for injecting an ejection gas.

A relative displacement between the stems is able to open a gap between the stems, for injecting an ejection gas for ejecting the moulded shell, the advantages of which have been described and are described in more detail below.

In an embodiment, said gap is provided along a line extending around said bulbous element.

In an embodiment, said gap is a continuous circular gap.

In an embodiment, each of the stems comprises a conical adjustment surface for mating with the other stem conical adjustment surface and forming said continuous circular gap between the conical adjustment surfaces.

In an embodiment, the mould core is configured such that the gap is sealed when the stems are displaced to the closest relative position between said stems.

In an embodiment, the first stem is a static stem and the second stem is a moveable stem relative to the static stem.

In an embodiment, said mould comprises a mould plate upon which each mould half is slidably attached for mating with the other mould half.

In an embodiment, the static stem is attached to the mould plate.

In an embodiment, the first portion of the stalk forms an outer stalk portion and the second portion of the stalk forms an inner stalk portion, arranged such that the inner stalk portion is slidable within the outer stalk portion.

In an embodiment, the mould core comprises a fluid-tight ejection gas channel between the outer stalk portion and the inner stalk portion for feeding ejection gas to the gap.

In an embodiment, the mould core comprises a texturized surface on the bulbous element, in particular a texturized surface on the first stem, on the second stem, or on both first and second stems.

In an embodiment, the mould cavity comprises a texturized surface.

In an embodiment, the mould comprises an injection gate arranged between the mould halves and opposite the stalk relative to the bulbous element for injecting the injection material for the shell, in particular comprising a runner system tangent to a distal end of the shell to be injected.

In an embodiment, the mould comprises a cold runner system for direct gating on the shell.

In an embodiment, the mould comprises one or more vacuum channels for extracting cure fumes.

In an embodiment, said gap is discontinuous and comprises multiple openings arranged along a line extending around said bulbous element.

In an embodiment, the mould halves are sliding shaping inserts, in particular sliding shaping inserts comprising linear sliding guides fixed on a mould plate.

In an embodiment, the mould comprises heaters inside the mould halves, in particular comprising additional heaters in a mould plate.

In an embodiment, the mould comprises thermal insulation plates for thermal equilibrium.

In an embodiment, the mould comprises a plurality of thermal probes for monitoring local temperatures in the mould.

In an embodiment, the mould comprises a water-cooling channel for avoiding cure of the material before injection.

In an embodiment, the mould is configured for an abrupt temperature difference between a cooled injection side and a heated ejection side of the mould.

It is also disclosed a moulding method for moulding a flexible implant shell for use in body implants using a mould according to any of the disclosed embodiments, comprising:

> injecting an injection material to said mould;
> curing said injection material by temperature and/or chemical effect;
> opening said mould halves sufficiently for removing the moulded shell;
> displacing the stems relative to each other, for opening said gap at the bulbous element between the first stem and the second stem;
> injecting through said gap the ejection gas for ejecting the moulded shell.

It is also disclosed a flexible implant shell for use in body implants moulded by any of the disclosed methods.

It is also disclosed a flexible implant shell for use in body implants moulded using any of the disclosed mould embodiments.

It is also disclosed a body implant comprising the flexible implant shell according to the disclosed embodiments.

In an embodiment, said body implant is a breast implant.

## BRIEF DESCRIPTION OF THE DRAWINGS

The following figures provide preferred embodiments for illustrating the disclosure and should not be seen as limiting the scope of invention.

> **Figure 1:** Schematic representation of an embodiment of the mould segments involved on the fabrication process of the said flexible implant shell.

> **Figure 2A:** Schematic cross section representation of an embodiment of the integration of the mould segment involved on the injection and curing phases and the outer structure of the mould - mould closed.

> **Figure 2B:** Schematic cross section representation of an embodiment of the integration of the mould segment involved on the ejection phase and the outer structure of the mould - mould open.

**Figure 3:** Schematic cross section view of an embodiment of the air gas connection to the air gas channel.

**Figure 4:** Schematic representation of an embodiment of the sliding system without cross sections.

**Figure 5A and B:** Photographic illustrations of a result analysis of thickness distribution on the obtained flexible shell by Computer Tomography of an embodiment of the disclosure.

**DETAILED DESCRIPTION**

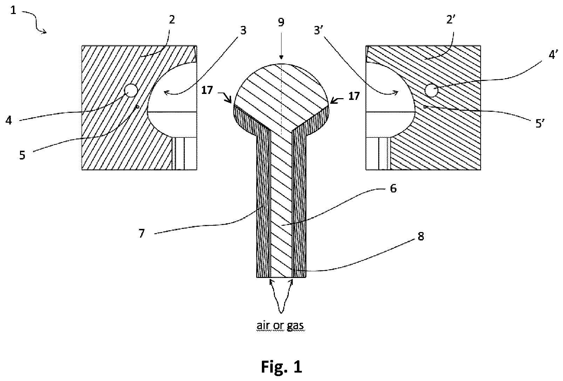

**Figure 1** shows a schematic representation of the mould 1 segments involved on the fabrication process of the said flexible implant shell. The mould 1 structure follows the normal composition of an injection moulding tool (not shown). The cross section shows in detail the simplified representation of the sliding elements of the mould **1** that shape the external geometry of the implant. This segment comprises two mould halves, see sliding shaping inserts **2** and **2'**, where the mould cavity **3, 3'** is placed, which are attached to main sliding blocks (not shown). The correct alignment of these cavities is preferably given by linear sliding guides fixed for example on a mould plate. The correct thermal distribution across the cavity is possible due to the presence of heaters **4** and **4'** inside the sliding shaping inserts **2** and **2'**. These heaters are considered to work under electrical resistances or induction. The thermal equilibrium in this region is obtained with the presence of several thermal insulation plates that cover these areas (not shown) and thermal probes **5** and **5'** are included to monitor the local temperature.

According to the disclosure, in order to fabricate a controlled flexible implant shell by injection moulding, the mould comprises two concavities, see lateral cavities **3** and **3'**, one of each sliding shaping inserts **2** and **2'** comprising a concave geometry that defines the external surface of the shell implant. The mould also comprises a core with a convex, i.e. bulbous, shape, which is formed by a "dynamic", i.e. movable, stem **6** and a static stem **7**, that defines the internal shape of the shell. This convex geometry shapes the inside surface of the shell. The convex shape of the mould core is supported on a stalk. This stalk is formed by both the dynamic stem **6** and the static stem **7**. High precision steel machining technologies are preferably used in order to guarantee small tolerances and the desired thickness distribution across all the geometry. Between the external diameter of dynamic stem **6** and the internal diameter of the static stem **7** there is a peripheral gap **17**, typically a circular peripheral gap, that forms the ejection gas channel **8**. This ejection gas channel **8** is blocked during the injection and curing phases of the injected material. The disclosed embodiment leads to the creation of a slight principal parting line **9** on the outer diameter surface of the flexible shell, coincident to the adjustment of the two sliding shaping inserts **2** and **2'**, represented by the dashed line.

Mould **1** may preferably be correctly placed and aligned on any conventional injection moulding machine.

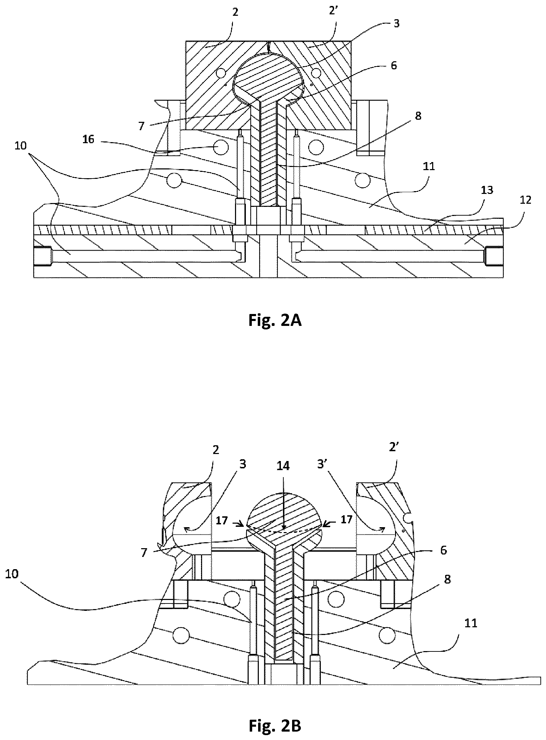

**Figures 2A** and **2B** show a cross section representation of the integration of the mould segment involved on the injection and curing phases and the outer structure of the mould that integrates the vacuum system **10** where it is shown the mould plate **11**, followed by back plate **12** and other structures like spacer blocks and a bottom clamp plate, as common seen in injection moulds design (not shown). Further in this **Figure 2,** it is shown the entrance for the vacuum system **10** which extends to the core, through the mould plate **11**. The dynamic internal stem **6** slides inside the static stem **7**, remaining between them an ejection gas channel **8**, which is sealed. Thermal insulation on the critical areas are preferably guaranteed by thermal insulation plates **13** between the mould plate **11** and the back plate **12**, as well as on the lateral of these plates and between the bottom clamp plate and the machine (not shown). The dynamic internal stem **6** is preferably part of the mould core together with the static stem **7**. **Figure 2A** shows the closed stage of the mould, correspondent to the injection and curing phases. **Figure 2B** shows the opening stage of the mould on the ejection phase. Besides that, there is a second mould parting line **14** formed because of the conical adjustment between the dynamic internal stem **6** the static stem **7**.

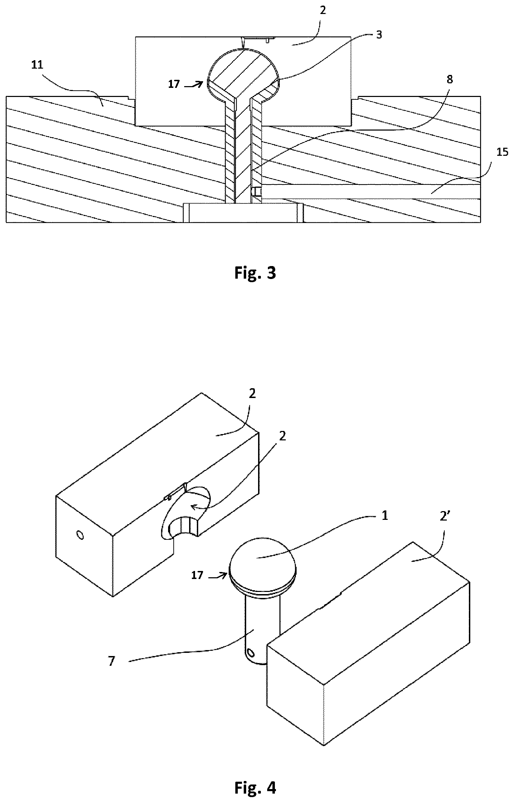

**Figure 3** shows a schematic cross section view of the air gas connection **15** to the air gas channel **8** through the mould plate **11**, perpendicular to the vacuum system **10**.

**Figure 4** shows a schematic representation of the system without cross sections.

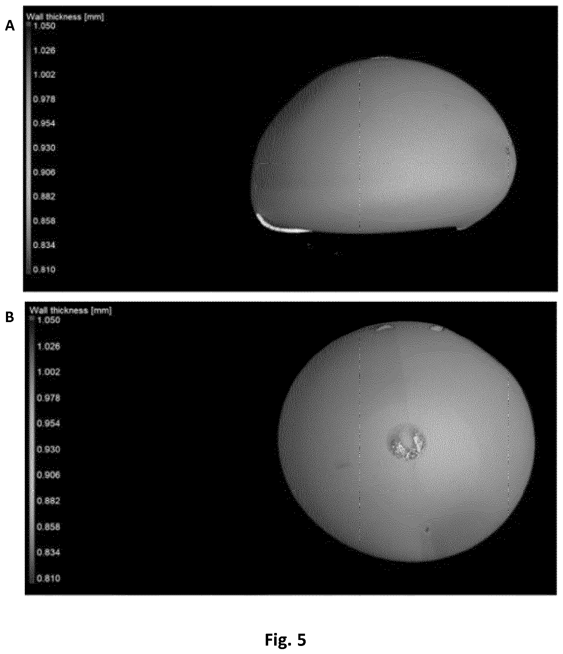

**Figures 5A** and **5B** show some result analysis of thickness distribution on the obtained flexible shell, from a lateral view parallel to the main parting line **9 (A)** and from the top view **(B)**.

For this disclosure, any fluid material under pressure during the injection of it in the mould that cures or solidifies on the core but behaves as a flexible material with enough elongation capacity to overcome the positive geometry of the core during ejection phase can be used. For the application purpose of breast implants, liquid silicone rubber materials are the preferable materials.

In order to speed up the curing and moulding process, the core and cavity elements of the mould must preferably achieve a balanced temperature range from 160 to 200°C.

However, pursuant to the heating curable materials special attention must preferably be given to the temperature of the fluid on the material injector in order to avoid premature curing of the material at this location, blocking the entrance of material into the mould 1. To avoid this, a water-cooling channel is preferably included around the material injector and across the injection plate (not shown). This area is preferably monitored by thermal probes in order to guarantee the desired temperature, ideally lower than 25°C.

According to an embodiment, due to an abrupt temperature difference between the injection side of the mould and the ejection side of the mould, a thermal insulation plate is preferably located on the contact region between injection plate and the top surface of the sliding shaping inserts **2** and **2'**.

In order to guarantee the correct filling, the gate is preferably located between the sliding shaping inserts **2** and **2'** according the traditional runner system, and is tangent to the top surface of the shell, allowing a radial and controlled filling in every direction. For the purpose of this disclosure, a cold runner system can also be used, allowing direct gating on the shell and avoiding waste of material, that in case of liquid silicone rubbers, or other thermosetting materials, is typically not recyclable.

Another important characteristic is related to the correct thermal distribution in order to maintain a fully automatic processing. To do so, several heating zones are preferably created with the right quantity of heaters **16** and controllers, connected to a self-tuning software linked to the injection moulding machine that controls and monitors the heating profile per area. Temperature will only accelerate the curing chemical reacting, meaning that this disclosure may also perform the curing without temperature, depending on the chemical properties of the injection material.

This disclosure also allows the implant surface to have texture, on both internal and external portions or just in one surface. It was considered that, in case of texturized surface implant is needed, the application of texture can be performed only on the dynamic **6** and static stem cores **7** surfaces. By turning the texturized surface to the opposite position (inside-out), the implant would have the texture surface on the exterior surface. This could also decrease the implant cost. In this disclosure, texture can also be applied to improve the ejection of the part. Surface texture on the mould surface can be used to decrease the liquid silicone adhesion to the steel, improving the ejection phase and decreasing the risk of shell rupture. In this case, it will not be necessary to turn the texturized surface to the opposite position (inside-out).

Besides the optional texture of the core surface, the ejection of the implant shell is supported by a mechanical displacement between the stems and air inflation on the cavity, towards the convex bulbous core formed by a dynamic stem **6** and a static stem **7**. In order to allow the ejection phase, the injection moulding machine removes the clamping force on the mould **1** and allows its opening. Preferably, when the mould **1** opens, the injection side of the mould remains static, while the ejection side of the mould is displaced until a safety distance for the complete opening of the sliding shaping elements **2** and **2'** to the sides is achieved and to allow the necessary displacement of the dynamic stem **6** on the normal direction, this is the ejection direction. The lateral opening of the sliding shaping elements **2** and **2'** is preferably guided by two angle pins. During the displacement of the sliding shaping elements **2** and **2'** the correct position is controlled by sliding guides.

After the preferable total opening displacement of the sliding shaping elements **2** and **2'**, the injection moulding machine applies an ejection course that actuates the main stem which in turn actuates the ejection plates (not shown). When these plates displace on the ejection direction, the internal dynamic stem **6** is also displaced. The displacement of the dynamic internal stem **6** occurs inside a static stem **7,** which is also part of the core. The internal diameter of the static stem **7** is preferably slightly larger than the external diameter of the dynamic internal stem **6,** allowing the creation of an ejection gas channel **8.** While the mould **1** is closed, during the injection and curing phases, until the ejection phase, this ejection gas channel **8** is completely closed, avoiding leakage of material inside the dual core or the even flash. A tenuous parting line **14** can be found in this stem adjustment surface, without negative impact for the final product. When the ejection phase starts, the displacement of the dynamic internal stem **6** allows the opening of the ejection gas channel **8,** meaning there is a peripheral channel up to the cavity and a small deformation elongation of the flexible shell still attached to the core.

In order to eject the implant shell more effectively and under a fully automatic process, during the ejection phase, a gas, meaning any kind, like simple air, nitrogen, carbon dioxide, or other, is injected inside the mould **1** through the mould plate **11** up to the ejection gas channel **8,** not shown. Since the ejection gas channel is open up to the shell cavity, the shell begins to be inflated radially on the major diameter region achieving a balloon-like configuration, until it is completely ejected to a handling that stores the shell, not shown.

Advantageously, this peripheral profile of inflation is of great interest since it decreases the local stress on the flexible shell caused by local elongations of the material.

It is not possible to ensure a completely spherical holed and closed geometry by this type of technology due to the stalk of the mould core. Thus, the disclosure allows a spherical holed geometry with an interruption caused by the stalk of the static stem 7 that forms the core. In this disclosure, this interruption may be used as an advantage for the product, since this method intends the fabrication a flexible implant shell that can be filled with materials that confer the desired volume and consistency to the implant, such as saline solutions, hydrogels, gels or others. This interruption can allow the entrance of such filling materials that can be later sealed with a lid of the same material of the shell, or other compatible material, through gluing, welding or other adhesion techniques.

The ejection of the flexible shell ends when the peripheral material surrounding shell hole (caused by the stalk of the static stem **7**) completely passes the maximum external diameter of the core formed by the dynamic stem **6** and the static stem **7**.

Advantageously this disclosure turns possible the real improvement of thickness control comparing to the traditional techniques used to produce the state of the art breast implant shells. For instance, thickness studies calculated according the following formula, with thickness data obtained by Computer Tomography, as shown on **Figures 5A** and **5B**:

$$Thickness\ variation\ (\%) = \frac{(Th_{max} - Th_{min})}{\overline{Th}} \times 100$$

Where $Th_{max}$ is the maximum thickness per implant, $Th_{min}$ is the minimum thickness per implant and $Th$ is the mean thickness per implant, revealed that commercial products present a thickness variation between 30.9 and 65.2%. The literature has also shown a strong correlation between thickness and stress, referring that an increase of thickness may lead to a stress increase, while lower thickness shows less stress. This absence of homogeneity is proven to be one of the main causes for the implant rupture.

Embodiments of the present disclosure have shown a thickness variation of 19%, which is 38% lower comparing to the state of the art products on the market.

Advantageously, the method can be aided by one or more robotic machines, in particular aided by a robot arranged for manipulating the moulded product after ejection from the mould.

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and its scope is defined by the appended claims.

**Claims**

1. Mould (1) for injection moulding a flexible implant shell for use in body implants, comprising:

   a mould core having a stalk and a convex bulbous element; and
   two mould halves (2, 2'), wherein each mould half is arranged for mating with the other mould half at respective confronting surfaces, each surface comprising a concavity (3,3') forming, in the mould halves, a mould cavity for mating with the bulbous element;
   **characterised in that** said mould core comprises:

   a first stem (7) forming a first portion of the stalk and a proximal portion of the convex bulbous element; and
   a second stem (6) forming a second portion of the stalk and a distal portion of the convex bulbous element; arranged such that one of the stems is displaceable relative to the other stem for opening a gap (17) at the bulbous element, between the first stem (7) and the second stem (6), for injecting an ejection gas for ejecting the moulded shell.

2. Mould (1) according to the previous claim wherein said gap (17) is provided along a line extending around said bulbous element.

3. Mould (1) according to any of the previous claims wherein said gap (17) is a continuous circular gap.

4. Mould (1) according to the previous claim wherein each of the stems (6,7) comprises a conical adjustment surface for mating with the other stem conical adjustment surface and for forming said continuous circular gap (17) between the conical adjustment surfaces.

5. Mould (1) according to any of the previous claims wherein the mould core is configured such that the gap (17) is sealed when the stems (6,7) are displaced to the closest relative position between said stems.

6. Mould (1) according to any of the previous claims wherein the first stem is a static stem (7) and the second stem (6) is a moveable stem relative to the static stem.

7. Mould (1) according to any of the previous claims wherein said mould comprises a mould plate upon which each mould half is slidably attached for mating with the other mould half.

8. Mould (1) according to the previous claim wherein the static stem is attached to the mould plate.

9. Mould (1) according to any of the previous claims wherein the first portion of the stalk forms an outer stalk portion and the second portion of the stalk forms an inner stalk portion, arranged such that the inner stalk portion is slidable within the outer stalk portion.

10. Mould (1) according to the previous claim wherein the mould core comprises a fluid-tight ejection gas channel (8) between the outer stalk portion and the inner stalk portion for feeding ejection gas to the gap (17).

11. Mould (1) according to any of the previous claims wherein the mould core comprises a texturized surface on the bulbous element, in particular a texturized surface on the first stem (7), on the second stem (6), or on both first and second stems.

12. Mould (1) according to any of the previous claims wherein the mould cavity (3) comprises a texturized surface.

13. Moulding method for moulding a flexible implant shell for use in body implants using a mould, comprising:

   injecting an injection material to said mould;
   curing said injection material by temperature and/or chemical effect;
   opening said mould halves (2,2') sufficiently for removing the moulded shell;
   **characterised in that** said mould is a mould (19) according to any of the previous claims, the method further comprising the steps of:

   displacing the stems (6,7) relative to each other, for opening said gap (17) at the bulbous element between the first stem (7) and the second stem (6);
   injecting through said gap (17) the ejection gas (8) for ejecting the moulded shell.


**Patentansprüche**

1. Form (1) zum Spritzgießen einer flexiblen Implantathülle zur Verwendung bei Körperimplantaten, umfassend:

   einen Formkern mit einem Stiel und einem konvexen, bauchigen Element; und
   zwei Formhälften (2, 2'), wobei jede Formhälfte so angeordnet ist, dass sie mit der anderen Formhälfte an jeweils gegenüberliegenden Oberflächen zusammenpasst, wobei jede Oberfläche eine Konkavität (3, 3') aufweist, die in den Formhälften einen Formhohlraum bilden, der mit dem bauchigen Element zusammenpasst;
   **dadurch gekennzeichnet, dass** der genannte Formkern umfasst:

   einen ersten Schaft (7), der einen ersten Teil des Stiels und einen proximalen Teil des konvexen, bauchigen Elements bildet; und
   einen zweiten Schaft (6), der einen zweiten Teil des Stiels und einen distalen Teil des konvexen, bauchigen Elements bildet;
   so angeordnet, dass einer der Schäfte relativ zu dem anderen Schaft versetzbar ist, um einen Spalt (17) an dem bauchigen Element zwischen dem ersten Schaft (7) und dem zweiten Schaft (6) zu öffnen, um ein Ausstoßgas zum Ausstoßen der geformten Hülle einzupressen.

2. Form (1) nach dem vorangehenden Anspruch, wobei der genannte Spalt (17) entlang einer Linie um das genannte bauchige Element herum verläuft.

3. Form (1) nach einem der vorangehenden Ansprüche, wobei der genannte Spalt (17) ein durchgehender kreisförmiger Spalt ist.

4. Form (1) nach dem vorangehenden Anspruch, wobei jeder der Schäfte (6, 7) eine konische Anpassfläche für das Zusammenpassen mit der konischen Anpassfläche des anderen Schaft aufweist, um den genannten kreisförmigen Spalt (17) zwischen den konischen Anpassflächen zu bilden.

5. Form (1) nach einem der vorangehenden Ansprüche, wobei der Formkern so gestaltet ist, dass der Spalt (17) abgedichtet ist, wenn die Schäfte (6, 7) in die engste relative Position zwischen den genannten Schäften verschoben werden.

6. Form (1) nach einem der vorangehenden Ansprüche, wobei der erste Schaft ein statischer Schaft (7) und der zweite Schaft (6) ein relativ zu dem statischen Schaft beweglicher Schaft ist.

7. Form (1) nach einem der vorangehenden Ansprüche, wobei die genannte Form eine Formplatte umfasst, auf der jede Formhälfte verschiebbar angebracht ist, um mit der anderen Formhälfte zusammenzupassen.

8. Form (1) nach dem vorangehenden Anspruch, wobei der statische Schaft an der Formplatte befestigt ist.

9. Form (1) nach einem der vorangehenden Ansprüche, wobei der erste Teil des Stiels einen äußeren Stielteil und der zweite Teil des Stiels einen inneren Stielteil bildet, die so angeordnet sind, dass der innere Stielteil innerhalb des äußeren Stielteils verschiebbar ist.

10. Form (1) nach dem vorangehenden Anspruch, wobei der Formkern einen flüssigkeitsdichten Ausstoßgaskanal (8) zwischen dem äußeren Stielteil und dem inneren Stielteil für die Zufuhr von Ausstoßgas in den Spalt (17) aufweist.

11. Form (1) nach einem der vorangehenden Ansprüche, wobei der Formkern eine texturierte Oberfläche auf dem bauchigen Element aufweist, insbesondere eine texturierte Oberfläche auf dem ersten Schaft (7), auf dem zweiten Schaft (6) oder auf beiden, dem ersten und dem zweiten Schaft.

12. Form (1) nach einem der vorangehenden Ansprüche, wobei der Formhohlraum (3) eine texturierte Oberfläche umfasst.

13. Formverfahren zum Formen einer flexiblen Implantathülle zur Verwendung bei Körperimplantaten unter Verwendung einer Form, umfassend:

Einspritzen eines Injektionsmaterials in die genannte Form;
Aushärten des Injektionsmaterials durch Temperatur und/oder chemische Wirkung;
Ausreichendes Öffnen der genannten Formhälften (2, 2'), um die geformte Hülle zu entfernen;
**dadurch gekennzeichnet, dass** die genannte Form eine Form (19) nach einem der vorangehenden Ansprüche ist, wobei das Verfahren ferner die folgenden Schritte umfasst:

Verschieben der Schäfte (6, 7) relativ zueinander, um den genannten Spalt (17) an dem bauchigen Element zwischen dem ersten Schaft (7) und dem zweiten Schaft (6) zu öffnen;
Einpressen des Ausstoßgases (8) durch den Spalt (17), um die geformte Hülle auszustoßen.

**Revendications**

1. Moule (1) pour moulage à injection d'une enveloppe d'implant flexible pour une utilisation dans les implants corporels, comprenant :

un noyau de moule ayant une tige et un élément bulbeux convexe ; et
deux moitiés de moule (2, 2'), dans lequel chaque moitié de moule est arrangée pour s'accoupler à l'autre moitié de moule au niveau de leurs surfaces se faisant face respectives, chaque surface comprenant un creux (3, 3') formant, dans les moitiés de moule, une cavité de moule pour s'accoupler à l'élément bulbeux ;
**caractérisé en ce que** ledit noyau de moule comprend :

une première tige (7) formant une première partie de la tige et une partie proximale de l'élément bulbeux convexe ; et
une seconde tige (6) formant une seconde partie de la tige et une partie distale de l'élément bulbeux convexe ;
arrangées telles que l'une des tiges soit déplaçable par rapport à l'autre tige pour ouvrir un écart (17) au niveau de l'élément bulbeux, entre la première tige (7) et la seconde tige (6), pour injecter un gaz à éjection pour éjecter l'enveloppe moulée.

**2.** Moule (1) selon la revendication précédente, dans lequel ledit écart (17) est prévu le long d'une ligne qui s'étend autour dudit élément bulbeux.

**3.** Moule (1) selon l'une quelconque des revendications précédentes, dans lequel ledit écart (17) est un écart circulaire continu.

**4.** Moule (1) selon la revendication précédente, dans lequel chacune des tiges (6, 7) comprend une surface d'ajustement conique pour s'accoupler à l'autre surface d'ajustement conique de tige et pour former ledit écart circulaire continu (17) entre les surfaces d'ajustement coniques.

**5.** Moule (1) selon l'une quelconque des revendications précédentes, dans lequel le noyau de moule est configuré tel que l'écart (17) soit scellé lorsque les tiges (6, 7) sont déplacées vers la position relative la plus proche entre lesdites tiges.

**6.** Moule (1) selon l'une quelconque des revendications précédentes, dans lequel la première tige est une tige statique (7) et la seconde tige (6) est une tige mobile par rapport à la tige statique.

**7.** Moule (1) selon l'une quelconque des revendications précédentes, dans lequel ledit moule comprend une plaque de moulage sur laquelle chaque moitié de moule est fixée de manière coulissante pour s'accoupler à l'autre moitié de moule.

**8.** Moule (1) selon la revendication précédente, dans lequel la tige statique est fixée à la plaque de moulage.

**9.** Moule (1) selon l'une quelconque des revendications précédentes, dans lequel la première partie de la tige forme une partie de tige extérieure et la seconde partie de la tige forme une partie de tige intérieure, arrangée telle que la partie de tige intérieure soit coulissante au sein de la partie de tige extérieure.

**10.** Moule (1) selon la revendication précédente, dans lequel le noyau de moule comprend un canal de gaz à éjection étanche au liquide (8) entre la partie de la tige extérieure et la partie de la tige intérieure pour mener le gaz à éjection jusqu'à l'écart (17).

**11.** Moule (1) selon l'une quelconque des revendications précédentes, dans lequel le noyau de moule comprend une surface texturisée sur l'élément bulbeux, en particulier une surface texturisée sur la première tige (7), sur la seconde tige (6), ou sur la première et la seconde tige.

**12.** Moule (1) selon l'une quelconque des revendications précédentes, dans lequel la cavité du moule (3) comprend une surface texturisée.

**13.** Procédé de moulage pour mouler une enveloppe d'implant flexible pour une utilisation dans les implants corporels utilisant un moule, comprenant :

  injecter un matériau d'injection dans ledit moule ;
  durcir ledit matériau d'injection par effet de température et/ou chimique ;
  ouvrir lesdites moitiés de moule (2, 2') suffisamment pour retirer l'enveloppe moulée ;
  **caractérisé en ce que** ledit moule est un moule (19) selon l'une quelconque des revendications précédentes, le procédé comprenant également les étapes de :

    déplacer les tiges (6, 7) l'une par rapport à l'autre, pour ouvrir ledit écart (17) au niveau de l'élément bulbeux entre la première tige (7) et la seconde tige (6) ;
    injecter le gaz à éjection (8) à travers ledit écart (17) pour éjecter l'enveloppe moulée.

**Fig. 1**

**Fig. 2A**

**Fig. 2B**

**Fig. 3**

**Fig. 4**

**Fig. 5**

**EP 3 718 508 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- FR 2734754
- WO 9718076 A
- US 2013171288 A
- US 2003134067 A

**15**